# EUROPEAN PATENT APPLICATION

(11) **EP 4 382 612 A1**
(43) Date of publication of application: **12.06.2024**
(21) Application number: 22853209.9
(22) Date of filing: 08.08.2022
(51) Int. Cl.: C12Q 1/02, C07K 14/705, C12N 5/10, C12N 15/12, G01N 33/02, G01N 33/50

(54) **METHOD FOR MEASURING RESPONSE OF OLFACTORY RECEPTOR TO SUBSTANCE**

(30) Priority: 06.08.2021 JP 2021129744
(71) Applicant: AJINOMOTO CO., INC., Chuo-ku Tokyo 104-8315 (JP)
(72) Inventor: IJICHI, Chiori, Kawasaki-shi, Kanagawa 210-8681 (JP); IHARA, Yusuke, Kawasaki-shi, Kanagawa 210-8681 (JP); SOMEKAWA, Yasuko, Kawasaki-shi, Kanagawa 210-8681 (JP); KAWATO, Yayoi, Kawasaki-shi, Kanagawa 210-8681 (JP); KARAKAWA, Sachise, Kawasaki-shi, Kanagawa 210-8681 (JP)
(74) Representative: Hoffmann Eitle
(86) International application number: PCT/JP2022/030329
(87) International publication number: WO 2023/013791

(57) **Abstract**

A method for measuring a response of an olfactory receptor to a substance is provided. The test substance and the olfactory receptor are contacted under such a condition that response of adenosine receptor to the test substance is reduced, and response of the olfactory receptor to the test substance is measured.

## Description

### Technical Field

The present invention relates to a method for measuring a response of an olfactory receptor to a substance.

### Background Art

Aromas are important elements that determine the palatability of foods, cosmetics, and other products. Therefore, technologies for screening aroma ingredients necessary to reproduce specific aromas, technologies for reproducing specific aromas by combining aroma ingredients, or technologies for screening ingredients that suppress (i.e., mask) undesirable specific aromas are industrially important for the development of foods, cosmetics, etc.

Conventionally, screening for ingredients that affect specific aroma has been conducted by humans by evaluating aromas of test substances through sensory testing. However, sensory testing has problems such as the need to train experts who can evaluate aromas, and low throughput.

In mammals such as humans, aromas are recognized thorough such events that odorant molecules bind to olfactory receptors on olfactory nerve cells in the olfactory epithelium of the upper nasal cavity, and responses of the receptors to the molecules are transmitted to the central nervous system. Specifically, it is known that when the olfactory receptors are activated by aroma ingredients, the olfactory receptors couple with intracellular G proteins (specifically, Gs such as Golf) to activate adenylate cyclase, thereby increasing the amount of intracellular cAMP (Non-patent document 1). In recent years, methods for screening substances that produce specific aromas or suppress specific aromas using olfactory receptor responses as an indicator have been reported (e.g., Patent documents 1 to 4, etc.).

Adenosine receptors are G protein-coupled receptors (GPCRs) that are responsive to adenosine. The adenosine receptors A1, A2A, A2B, and A3 are known as the adenosine receptors. The adenosine receptors A2A and A2B are known as adenosine receptors that couple with Gs protein.

### Prior Art References

### Patent documents

Patent document 1: Japanese Patent Publication (Kokai) No. 2019-037197
Patent document 2: WO2021/064201
Patent document 3: Japanese Patent Publication (Kokai) No. 2019-129773
Patent document 4: Japanese Patent Publication (Kokai) No. 2019-129772

### Non-patent document

Non-patent document 1: Kajiya K. et al, Molecular bases of odor discrimination: Reconstitution of olfactory receptors that recognize overlapping sets of odorants, Journal of Neuroscience, 2001, 21:6018-6025

### Summary of Invention

### Object to be achieved by the invention

An object of the present invention is to provide a method for measuring a response of an olfactory receptor to a substance.

### Means for achieving the object

The inventors of the present invention found that, when measuring responses of olfactory receptors to test substances such as foods, nonspecific responses (i.e., responses of olfactory receptors other than those to the test substances) can be observed, and that use of adenosine receptor antagonists can reduce such nonspecific responses, thereby enabling specific measurement of olfactory receptor responses to the test substances, and thus accomplished the present invention.

That is, the present invention can be embodied, for example, as follows
[1] A method for measuring a response of an olfactory receptor to a test substance comprising the following steps (A) and (B):
   (A) a step of contacting the olfactory receptor with the test substance; and
   (B) a step of measuring the response of the olfactory receptor to the test substance, wherein the olfactory receptor is used in the form of being carried on a cell or on a cell membrane prepared from the cell, and
      wherein the step (A) is performed under the following condition (X):
      (X) a condition that a response of an adenosine receptor to the test substance is reduced.
[2] The method described above, wherein the adenosine receptor is adenosine receptor A2A and/or A2B.
[3] The method described above, wherein the condition (X) is achieved by performing the step (A) in the presence of an adenosine receptor antagonist.
[4] The method described above, wherein the adenosine receptor antagonist is selected from the group consisting of caffeine, theophylline, istradefylline, CGS-15943, SCH-58261, SCH-442416, ZM-241385, CVT-6883, MRS-1706, MRS-1754, MRE-2029-F20, PSB-603, PSB-0788, and PSB-1115.
[5] The method described above, wherein the adenosine receptor antagonist is used at a concentration of 0.05 to 1000 µM.
[6] The method described above, wherein the condition (X) is achieved because the cell have the following characteristic (X1) or (X2):
   (X1) the cell has been modified so that the activity of the adenosine receptor is reduced; or
   (X2) the cell does not inherently have the adenosine receptor.
[7] The method described above, wherein the cells have been modified so as not to have the adenosine receptor.
[8] The method described above, wherein the response of the olfactory receptor is activation of the olfactory receptor.
[9] The method described above, wherein the response of the olfactory receptor is inactivation of the olfactory receptor, and the step (A) is performed in the presence of a substance that activates the olfactory receptor.
[10] The method described above, wherein the olfactory receptor is used in the form of being carried on the cells.
[11] The method described above, wherein the cell is an animal cell.
[12] The method described above, wherein the response is measured by using intracellular cAMP concentration or intracellular calcium concentration as an indicator.
[13] The method described above, wherein the intracellular cAMP concentration is measured by a reporter assay.
[14] The method described above, wherein the test substance is selected from the group consisting of foods, raw materials thereof, and fractionation products thereof.
[15] The method described above, wherein the olfactory receptor is selected from the group consisting of OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K13, OR4K14, OR4K15, OR4K17, OR4L1, OR4M1, OR4N2, OR4N4, OR4N5, OR4P4, OR4Q3, OR4S1, OR4S2, OR4X1, OR4X2, ORSA1, OR5A2, OR5AC2, OR5AK2, OR5AK3P, OR5AN1, OR5AP2, ORSAR1, OR5AS1, OR5AU1, OR5B2, OR5B3, OR5B12, OR5B17, OR5B21, OR5C1, OR5D13, OR5D14, OR5D16, OR5D18, OR5F1, ORSH1, OR5H2, OR5H6, OR5H14, OR5I1, OR5J2, ORSK1, OR5K3, OR5K4, OR5L2, OR5M3, OR5M8, OR5M9, OR5M10, OR5M11, OR5P3, ORSR1, OR5T1, OR5T2, OR5T3, ORSV1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.
[16] The method described above, wherein the olfactory receptor is a human olfactory receptor.

### Brief Description of the Drawings

[Fig. 1] Responses of control cells (cells transfected with the empty vector Rho-pME18S) to adenosine.
[Fig. 2] Inhibition of responses of control cells (cells transfected with the empty vector Rho-pME18S) to adenosine by the adenosine receptor antagonist CGS-15943.
[Fig. 3] Responses of control cells (cells transfected with the empty vector Rho-pME18S) to the test substances.
[Fig. 4] Responses of olfactory receptor OR10G7-expressing cells to the test substances.
[Fig. 5] Correlation between the concentration of adenosine in the test substance and nonspecific responses of cells to the test substance.
[Fig. 6] Olfactory receptor activities (common logarithms of normalized responses) measured in the presence or absence of the adenosine receptor antagonist CGS-15943 using Hondashi^{(R)} Irikodashi as a test substance. Panel (A), results obtained in the absence of CGS-15943; Panel (B): results obtained in the presence of CGS-15943.
[Fig. 7] Olfactory receptor activities (common logarithms of normalized responses) measured in the presence or absence of the adenosine receptor antagonist CGS-15943 using Knorr^{(R)} Tsubutappuri Corn Cream as a test substance. Panel (A), results obtained in the absence of CGS-15943; Panel (B): results obtained in the presence of CGS-15943.

### Modes for Carrying out the Present Invention

Hereafter, the present invention will be explained in detail.

The method of the present invention is a method for measuring a response of an olfactory receptor to a test substance. The method of the present invention can be performed under a condition that the response of the adenosine receptor to the test substance is reduced. By carrying out the method of the present invention under a condition that the response of the adenosine receptor to the test substance is reduced, the response of the olfactory receptor to the test substance can be specifically measured. Specifically, the method of the present invention can be performed by contacting the test substance and the olfactory receptor under a condition that the response of the adenosine receptor to the test substance is reduced, and measuring the response of the olfactory receptor to the test substance. The adenosine receptor can be cells or cell membranes that carry the olfactory receptor. That is, the method of the present invention may specifically be a method for measuring a response of an olfactory receptor to a test substance comprising the steps of (A) contacting the olfactory receptor and the test substance; and (B) measuring the response of the olfactory receptor to the test substance, wherein the olfactory receptor is used either in the form of being carried on cells or cell membranes prepared from the cells, and the step (A) is performed under a condition that the response of the adenosine receptor to the test substance is reduced.

### <1> Test substance

The term "test substance" refers to a substance for which response of the olfactory receptor is measured in the method of the present invention. The test substance is not particularly limited so long as the test substance can elicit a response of adenosine receptor. The response of adenosine receptor includes activation of the adenosine receptor. The test substance may consist of a single ingredient or a combination of two or more types of ingredients. When the test substance consists of a combination of two or more types of ingredients, the number of types and composition ratio of the ingredients that constitute the combination are not particularly restricted. The test substance may be a known substance or a novel substance. The test substance may be a natural or artificial substance. The test substance may be, for example, a compound library produced by using combinatorial chemistry techniques. Examples of the test substance include substances that can contain an adenosine receptor agonist, such as adenosine, and substances that can be an adenosine receptor agonist. As the test substance, in particular, substances that can contain adenosine can be mentioned. That is, as the test substance, for example, and in particular, such a test substance as described below that can contain adenosine can be selected. Specific examples of the test substance include foods, cosmetics, raw materials thereof, and fractionation products (e.g., distillates) thereof. Examples of the test substance include, in particular, foods, raw materials thereof, and fractionation products (e.g., distillates) thereof. The foods include beverages and seasonings. The foods include drinks such as coffee drinks, black tea drinks, green tea drinks, lactic drinks, lactic acid bacteria drinks, soft drinks, carbonated drinks, fruit juice drinks, vegetable drinks, sports drinks, jelly drinks, powdered drinks, and alcoholic drinks; noodle soups such as udon soup, soba soup, somen soup, ramen soup, champon soup, and pasta sauce; soup stocks such as Japanese soup stock and chicken soup; seasonings such as soy sauce, fish sauce, Chinese sauce, oyster sauce, dressing, miso, mayonnaise, tomato ketchup, and creaming powder; soups such as egg soup, wakame soup, shark fin soup, Chinese soup, consomme soup, curry flavor soup, potage soup, corn soup, Japanese clear soup, and miso soup; and extracts such as beef extract, pork extract, and chicken extract. One food can also be a raw material of another food. Examples of cosmetics include cosmetics, perfumes, and toiletries. Specific examples of the test substance also include alcohols, ketones, aldehydes, ethers, esters, hydrocarbons, sugars, organic acids, nucleic acids, amino acids, peptides, and various other organic or inorganic ingredients. As specific examples of the test substance, existing food additives can be mentioned. The term "existing food additive" means a substance of which use as a food additive has already been approved. As the test substance, one kind of test substance may be used, or a combination of two or more kinds of test substances may be used.

### <2> Olfactory receptor

The olfactory receptor is not particularly limited. As the olfactory receptor, one type of olfactory receptor may be used, or a combination of two or more types of olfactory receptors may be used. When a combination of two or more types of olfactory receptors is used, the responses of the olfactory receptors to the test substance may be, for example, separately measured for each of them. For example, responses of olfactory receptors to a test substance can be separately measured by using cells expressing each of the olfactory receptors.

Examples of the olfactory receptors include OR1A1, OR1A2, OR1B1, OR1C1, OR1D2, OR1D4, OR1D5, OR1E1, OR1E2, OR1F1, OR1F12, OR1G1, OR1I1, OR1J1, OR1J2, OR1J4, OR1K1, OR1L1, OR1L3, OR1L4, OR1L6, OR1L8, OR1M1, OR1N1, OR1N2, OR1Q1, OR1R1P, OR1S1, OR1S2, OR2A1, OR2A2, OR2A4, OR2A5, OR2A7, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2F2, OR2G2, OR2G3, OR2G6, OR2H1, OR2H2, OR2J1P, OR2J2, OR2J3, OR2K2, OR2L2, OR2L3, OR2L5, OR2L8, OR2L13, OR2M2, OR2M3, OR2M4, OR2M5, OR2M7, OR2S2, OR2T1, OR2T2, OR2T3, OR2T4, OR2T5, OR2T6, OR2T7, OR2T8, OR2T10, OR2T11, OR2T12, OR2T27, OR2T29, OR2T33, OR2T34, OR2T35, OR2V1, OR2V2, OR2W1, OR2W3, OR2Y1, OR2Z1, OR3A1, OR3A2, OR3A3, OR3A4, OR4A4P, OR4A5, OR4A15, OR4A16, OR4A47, OR4B1, OR4C3, OR4C5, OR4C6, OR4C11, OR4C12, OR4C13, OR4C15, OR4C16, OR4C45, OR4C46, OR4D1, OR4D2, OR4D5, OR4D6, OR4D9, OR4D10, OR4D11, OR4E2, OR4F3, OR4F4, OR4F5, OR4F6, OR4F14P, OR4F15, OR4F17, OR4F21, OR4G11P, OR4H12P, OR4K1, OR4K2, OR4K5, OR4K13, OR4K14, OR4K15, OR4K17, OR4L1, OR4M1, OR4M2, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5AU1, OR5B2, OR5B3, OR5B12, OR5B17, OR5B21, OR5C1, OR5D13, OR5D14, OR5D16, OR5D18, ORSF1, OR5H1, OR5H2, OR5H6, OR5H14, OR5H15, OR5I1, OR5J2, OR5K1, OR5K2, OR5K3, OR5K4, OR5L1, OR5L2, OR5M1, OR5M3, OR5M8, OR5M9, OR5M10, ORSM11, OR5P2, OR5P3, ORSR1, OR5T1, OR5T2, OR5T3, ORSV1, OR5W2, OR6A2, OR6B1, OR6B2, OR6B3, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B2, OR8B3, OR8B4, OR8B8, OR8B12, OR8D1, OR8D2, OR8D4, OR8G1, OR8G2, OR8G5, OR8H1, OR8H2, OR8H3, OR8I2, OR8J1, OR8J3, OR8K1, OR8K3, OR8K5, OR8S1, OR8U1, OR8U8, OR9A2, OR9A4, OR9G1, OR9G4, OR9I1, OR9K2, OR9Q1, OR9Q2, OR10A2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G8, OR10G9, OR10H1, OR10H2, OR10H3, OR10H4, OR10H5, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H1, OR11H2, OR11H4, OR11H6, OR11H12, OR11L1, OR12D2, OR12D3, OR13A1, OR13C2, OR13C3, OR13C4, OR13C5, OR13C8, OR13C9, OR13D1, OR13F1, OR13G1, OR13H1, OR13J1, OR14A2, OR14A16, OR14C36, OR14I1, OR14J1, OR14K1, OR14L1P, OR51A1P, OR51A2, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E6, OR52E8, OR52H1, OR52I1, OR52I2, OR52J3, OR52K1, OR52K2, OR52L1, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.

Examples of the olfactory receptors include, in particular, OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K13, OR4K14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, ORSAR1, ORSAS1, OR5AU1, OR5B2, OR5B3, OR5B12, OR5B17, OR5B21, OR5C1, OR5D13, OR5D14, OR5D16, OR5D18, ORSF1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, ORSR1, OR5T1, OR5T2, OR5T3, ORSV1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.

A gene encoding an olfactory receptor is also referred to as "olfactory receptor gene".

Examples of the olfactory receptor gene and olfactory receptor include olfactory receptor genes and olfactory receptors of various organisms. Examples of the organisms include, for example, animals including mammals. Specific examples of mammals include, for example, Homo sapiens (human), Mus musculus (mouse), Rattus norvegicus (rat), Canis lupus familiaris (dog), Felis catus (cat), Bos taurus (cow), Sus scrofa (pig), Pan troglodytes (chimpanzee), Macaca fascicularis (crab-eating monkey), and Equus caballus (horse). As an example of the animals including mammals, in particular, human can be mentioned. Nucleotide sequences of olfactory receptor genes and amino acid sequences of olfactory receptors of various organisms can be obtained from public databases, for example, NCBI and Ensembl.

The olfactory receptor may be, for example, a protein having a known or natural amino acid sequence of any of such olfactory receptors as described above. The olfactory receptor may also be, for example, a conserved variant of a protein having a known or natural amino acid sequence of any of such olfactory receptors as described above. That is, the olfactory receptors identified by the above names shall encompass, for example, proteins having known or natural amino acid sequences of the olfactory receptors identified by the names and their conserved variants. The term "conserved variant" means a variant that maintains an original function. For the olfactory receptor, the expression "maintain an original function" can mean that a response of the olfactory receptor variant is elicited by a substance that elicits the response of the original olfactory receptor (e.g., a substance that activates the original olfactory receptor). The expression "protein has an amino acid sequence" means that the protein contains such an amino acid sequence, unless otherwise noted, and also means that the protein consists of such an amino acid sequence. Variants include proteins having an amino acid sequence derived from a known or natural amino acid sequence by substitution, deletion, insertion, and/or addition of one or several amino acids at one or more positions. The expression "one or several" may specifically mean a number of, for example, 1 to 50, 1 to 40, or 1 to 30, preferably 1 to 20, more preferably 1 to 10, still more preferably 1 to 5, especially preferably 1 to 3. Variants also include proteins having an identity of, for example, 50% or higher, 65% or higher, 80% or higher, preferably 90% or higher, more preferably 95% or higher, even more preferably 97% or higher, especially preferably 99% or higher, to the entire sequence of a known or natural amino acid sequence. The olfactory receptor identified by the species from which the olfactory receptor is derived is not limited to the olfactory receptor itself found in that species, but may also be a protein having the amino acid sequence of the olfactory receptor found in the species and variants thereof. Variants may or may not be found in that species. That is, for example, "human olfactory receptors" shall not limited to the olfactory receptors found in humans themselves, but include proteins having an amino acid sequence of an olfactory receptor found in humans and variants thereof. The olfactory receptor may be, for example, a chimeric protein of two or more types of olfactory receptors of different origins. That is, the olfactory receptors identified by each of the above names shall also include, for example, chimeric proteins of two or more types of olfactory receptors of different origins identified by those names.

The "identity" between amino acid sequences means identity between amino acid sequences calculated by blastp using the default settings of Scoring Parameters (Matrix, BLOSUM62; Gap Costs, Existence=11, Extension=1; Compositional Adjustments, Conditional compositional score matrix adjustment).

The olfactory receptor is used in the form of being carried on a cell or on a cell membrane prepared from such cell.

A cell having the olfactory receptor is also referred to as "a cell of the present invention". The olfactory receptor can, for example, localizes and functions in a cell membrane. Therefore, the cell of the present invention may have the olfactory receptor in, for example, a cell membrane.

Olfactory receptors are expressed from olfactory receptor genes. Therefore, the cell of the present invention has an olfactory receptor gene. Specifically, the cell of the present invention has an olfactory receptor gene in such a manner that the gene can be expressed in the cell. It is sufficient that the cell of the present invention has the olfactory receptor gene until it expresses the olfactory receptor. In other words, the cell of the present invention may or may not have the olfactory receptor gene after the expression of the olfactory receptor. Also in other words, the cell of the present invention is a cell that has expressed an olfactory receptor gene and expressed an olfactory receptor. The terms "expression of olfactory receptor gene" and "expression of olfactory receptor" can be synonymously used.

The cell of the present invention may have one copy of an olfactory receptor gene or two or more copies of the olfactory receptor gene.

The cell of the present invention may inherently have an olfactory receptor gene or may be modified to have the olfactory receptor gene.

Examples of the cell that inherently have an olfactory receptor gene include a cell of organism from which the above-mentioned olfactory receptor gene is derived, e.g., a taste cell of mammal such as human. A cell that inherently has an olfactory receptor gene can be obtained from, for example, an organism or a tissue that contains such cell.

A cell that has been modified to have an olfactory receptor gene include a cell into which the olfactory receptor gene has been introduced.

The cell of the present invention and a cell used to obtain it (e.g., a cell into which an olfactory receptor gene is to be or has been introduced) are also collectively referred to as "a host cell".

The host cell is not particularly limited so long as it can express an olfactory receptor that can function and can thus be used to measure a response of the olfactory receptor to a test substance. Examples of the host cell include, for example, bacterial cells, fungal cells, plant cells, insect cells, and animal cells. Preferred examples of the host cell include eukaryotic cells such as fungal cells, plant cells, insect cells, and animal cells. More preferred examples of the host cell include animal cells. The animals include, for example, mammals, birds, and amphibians. Examples of the mammals include, for example, rodents and primates. Examples of the rodents include Chinese hamster, hamster, mouse, rat, and guinea pig. Examples of the primates include human, monkey, and chimpanzee. Examples of the birds include, for example, chicken. Examples of the amphibians include, for example, African clawed frog. The tissue or cells from which the host cell is derived are not particularly limited. Examples of tissue or cells from which the host cell is derived include ovary, kidney, adrenal gland, lingual epithelium, olfactory epithelium, pineal gland, thyroid gland, and melanocytes. Examples of Chinese hamster cells include, for example, those of Chinese hamster ovary-derived cell lines (CHO). Specific examples of CHO include CHO-DG44 and CHO-K1. Examples of human cells include, for example, those of human embryonic kidney cell-derived cell lines (HEK). Specific examples of HEK include, for example, HEK293 and HEK293T. Examples of monkey cells include, for example, those of African green monkey kidney cell-derived cell line (COS). Specific examples of COS include, for example, COS-1. Examples of African clawed frog cells include, for example, African clawed frog oocytes. Examples of insect cells include, for example, Spodoptera frugiperda-derived cells such as Sf9, Sf21, and SF+ cells, and Trichoplusia niderived cells such as High-Five cells. The host cells may be independent individual cells (e.g., free cells) or may form aggregates such as tissues.

Olfactory receptor genes can be obtained by cloning from organisms that have the olfactory receptor genes. Nucleic acids such as genomic DNAs or cDNAs containing the genes can be used for cloning. Olfactory receptor genes can also be obtained by chemical synthesis (Gene, 60(1), 115-127 (1987)).

Obtained olfactory receptor genes can be used as they are or after being appropriately modified. That is, the olfactory receptor genes can be modified to obtain a variant thereof. The genes can be modified by known methods. For example, a site-specific mutagenesis method can be used to introduce a desired mutation at a target site of DNA. That is, for example, a coding region of a gene can be modified by the site-specific mutagenesis method so that the encoded protein contains substitution, deletion, insertion, and/or addition of amino acid residues at a specific site. Examples of the site-specific mutagenesis method include methods using PCR (Higuchi, R., 61, in PCR technology, Erlich, H.A. Eds., Stockton press (1989); Carter, P., Meth. in Enzymol., 154, 382 (1987)), and methods using phage (Kramer, W. and Frits, H.J., Meth. in Enzymol., 154, 350 (1987); Kunkel, T.A., et al. Meth. in Enzymol., 154, 367 (1987)). Variants of olfactory receptor genes may also be directly obtained by chemical synthesis.

The form of the olfactory receptor gene introduced into the host cell is not particularly limited. The olfactory receptor gene should be retained in the host cell so that it can be expressed. Specifically, for example, when the olfactory receptor gene is introduced in a form that requires transcription, such as DNA, it is sufficient that the olfactory receptor gene is maintained in the host cell so that it can be expressed under the control of a promoter that functions in the host cell. In the host cell, the olfactory receptor gene may be present outside the chromosome or introduced into the chromosome. When two or more types of genes are introduced, it is sufficient that each gene is retained in the host cell in such a manner that it can be expressed.

The promoter for expression of the olfactory receptor gene is not particularly limited so long as it can function in the host cell. The expression "a promoter that functions in host cell" means a promoter that shows promoter activity in the host cell. The promoter may be a promoter derived from the host cell or a promoter of heterologous origin. The promoter may be an intrinsic promoter of the olfactory receptor gene or a promoter of another gene. The promoter may be a more potent promoter than the intrinsic promoter of the olfactory receptor gene. Example of promoters that function in animal cells include, for example, SV40 promoter, EF1a promoter, RSV promoter, CMV promoter, and SRalpha promoter. In addition, by using various reporter genes, highly active forms of conventional promoters may be obtained and used as promoters. Methods for evaluating promoter strength and examples of strong promoters are described in the paper of Goldstein et al. (Prokaryotic promoters in biotechnology, Biotechnol. Annu. Rev., 1, 105-128 (1995)), and so forth.

The olfactory receptor gene can be introduced into host cell by using, for example, a vector containing the gene. A vector containing an olfactory receptor gene is also referred to as "olfactory receptor gene expression vector". The olfactory receptor gene expression vector can be constructed by, for example, ligating a DNA fragment containing an olfactory receptor gene with a vector. By introducing the olfactory receptor gene expression vector into the host cell, the gene can be introduced into the host cell. The vector may have a marker, such as a drug resistance gene. The vector may also have an expression control sequence such as a promoter for expressing an inserted gene. The vector can be appropriately selected according to various conditions, such as the type of host cell and the form of the olfactory receptor gene at the time of the introduction. Examples of vectors that can be used for gene transfer into animal cells include, for example, plasmid vectors and viral vectors. Examples of the viral vectors include, for example, retrovirus vectors and adenovirus vectors. Examples of the plasmid vectors include, for example, pcDNA series vectors (pcDNA3.1, etc., Thermo Fisher Scientific), pBApo-CMV series vectors (Takara Bio), and pCI-neo (Promega). Depending on the type and configuration of the vector, the vector can be incorporated into the host cell chromosome, autonomously replicate outside the host cell chromosome, or be temporarily retained outside the host cell chromosome. For example, vectors having a viral replication origin, such as SV40 replication origin, can autonomously replicate outside the chromosomes of animal cells. Specifically, for example, the pcDNA series vectors have the SV40 replication origin and can autonomously replicate outside the chromosome in host cells that express the SV40 large T antigen (such as COS-1 and HEK293T).

The olfactory receptor gene can also be introduced into the host cell by, for example, introducing a nucleic acid fragment containing the gene into the host cell. A nucleic acid fragment containing an olfactory receptor gene is also referred to as "olfactory receptor gene fragment". Examples of such a fragment include linear DNA and linear RNA. Examples of linear RNA include, for example, mRNA and cRNA.

The method for introducing a nucleic acid such as vector and nucleic acid fragment into the host cell can be selected according to various conditions such as the type of host cell. Examples of methods for introducing nucleic acids such as vectors and nucleic acid fragments into the host cell such as animal cells include, for example, the DEAE dextran method, calcium phosphate method, lipofection method, electroporation method, and microinjection method. When the vector is a viral vector, the vector can be introduced into the host cell by infecting the host cell with the vector (virus).

A cell that inherently has an olfactory receptor gene may also be modified so that the expression of the olfactory receptor gene is enhanced. The expression "expression of a gene is enhanced" means that the expression amount of the gene per cell is increased compared with unmodified cell. The term "unmodified cell" used here means a control cell that has not been modified to increase expression of a target gene. Examples of the unmodified cell include wild-type cells and cells from which the modified cells have been obtained. Techniques to increase expression of an olfactory receptor gene include increasing copy number of the olfactory receptor gene and improving transcription or translation efficiency of the olfactory receptor gene. Increasing copy number of an olfactory receptor gene can be achieved by introducing the olfactory receptor gene into the host cell. The introduction of the olfactory receptor gene can be performed as described above. The olfactory receptor gene to be introduced may be of host cell origin or heterologous origin. Improvement of transcription or translation efficiency of an olfactory receptor gene can be achieved by modifying an expression control sequence of the gene such as promoter. For example, improvement of the transcription efficiency of the olfactory receptor gene can be achieved by replacing the promoter of the olfactory receptor gene with a more potent promoter.

The cell of the present invention may have any other arbitrary properties so long as they can be used to measure a response of an olfactory receptor to a test substance. Such properties include, for example, properties useful for measuring a response of an olfactory receptor to a test substance.

The cell of the present invention may or may not have, for example, olfactory receptors other than the selected olfactory receptor (also referred to as "other olfactory receptors"). It may be desirable for the cell of the present invention to have no other olfactory receptors. A cell having no other olfactory receptors include a cell that does not have genes encoding other olfactory receptors or a cell that has genes encoding other olfactory receptors but does not express those genes. The cell of the present invention may be, for example, inherently devoid of other olfactory receptors or may be modified to be devoid of other olfactory receptors. Modifying a cell so that it does not have other olfactory receptors can be achieved by, for example, knocking out genes encoding other olfactory receptors.

The cell of the present invention may also have, for example, proteins involved in signal transduction. In other words, the cell of the present invention may have genes encoding proteins involved in signal transduction. Examples of proteins involved in signal transduction include, for example, G proteins (such as Golf), G protein activators (such as Ric8B), adenylate cyclase, and calcium channel proteins. Examples of Golf include, for example, animal Golf such as human Golf (GenBank accession No. NP_892023). Examples of Ric8B includes, for example, animal Ric8B such as rat Ric8B (GenBank accession No. NP_783188). The cell of the present invention may also have, for example, components appropriate for the parameters to be measured. Examples of such components include probes such as those for calcium indicators and reporter genes such as luciferase genes. When a probe for a calcium indicator or the like is expressed from a gene, the cell of the present invention may have a gene encoding such a probe.

The cell of the present invention may also have, for example, proteins that promote membrane expression of olfactory receptors. In other words, the cell of the present invention may have genes encoding such proteins. Examples of such proteins include, for example, RTP1s (Zhuang H. and Matsunami H., J. Biol. Chem., 282, 15284-15293 (2007)). Examples of RTP1s include, for example, animal RTP1s such as human RTP1s (GenBank accession No. AAT70680), mouse RTP1s (GenBank accession No. ABU23737), and bat RTP1s (amino acid sequence from the methionine residue at position 37 to the C-terminus of GenBank accession No. XP_006765914). The mouse RTP1s has 93.3% identity to the amino acid sequence of human RTP1s. The bat RTP1s (partial sequence mentioned above) has 90.7% identity to the amino acid sequence of human RTP1s.

The cell of the present invention may be a cell that inherently has such properties as exemplified above, or may be a cell modified to have such properties as exemplified above. For the modification of cell, the descriptions of cell modification concerning olfactory receptor genes, such as introduction of olfactory receptor genes, can be correspondingly applied. The genes exemplified above may be a gene derived from a host cell or a gene derived from a different species. The genes exemplified above may or may not be of the same origin as the olfactory receptor gene. When two or more genes are introduced, it is sufficient that each gene is retained in the host cell in such a manner that each gene can be expressed. These genes may be retained, for example, all on a single expression vector, or on a chromosome. These genes may also be retained, for example, separately on multiple expression vectors, or separately on a single or multiple expression vectors and on a chromosome. The genes exemplified above and the proteins encoded thereby may have, for example, nucleotide sequences and amino acid sequences of known genes and proteins, respectively. The genes exemplified above and protein encoded thereby may also be, for example, conserved variants of known genes and proteins, respectively. For conserved variants of the genes and proteins, the descriptions concerning conserved variants of olfactory receptor genes and olfactory receptors can be correspondingly applied.

The cell of the present invention may have the following characteristic (X1) or (X2):
(X1) the cell is modified so that the activity of the adenosine receptor is reduced; or
(X2) the cell does not inherently have the adenosine receptor.

Examples of the cells having the characteristic (X1) include a cell that has been modified so as not to have an adenosine receptor. That is, the characteristic (X1) means that, for example, the cell has been modified so as not to have an adenosine receptor. In other words, the cell of the present invention may be a cell that does not have an adenosine receptor, for example, a cell that has been modified so as not to have an adenosine receptor or a cell that does not inherently have an adenosine receptor. Examples of cells not having adenosine receptors include a cell that does not have any adenosine receptor gene and a cell that has an adenosine receptor gene but does not express that gene. Modifying a cell so that it does not have adenosine receptor can be achieved by, for example, knocking out (i.e., disrupting) the adenosine receptor gene.

The term "adenosine receptor" means a G protein-coupled receptor (GPCR) that is responsive to adenosine. The expression "responsive to adenosine" may mean to be activated by adenosine. The adenosine receptor may be, for example, an adenosine receptor that can couple with the Gs protein. Examples of the adenosine receptor include adenosine receptors A1, A2A, A2B, and A3. For example, the adenosine receptor is in particular, adenosine receptor A2A or A2B. The adenosine receptors A2A and A2B can be adenosine receptors that can couple with Gs protein. As the adenosine receptor, one kind of adenosine receptor may be selected, or two or more kinds of adenosine receptors may be selected. For example, adenosine receptors A2A and/or A2B may be selected as the adenosine receptor. For example, adenosine receptors A2A and A2B, in particular, may be selected as the adenosine receptor. A gene encoding an adenosine receptor is also referred to as "adenosine receptor gene". Nucleotide sequences of adenosine receptor genes and amino acid sequences of adenosine receptors of various organisms can be obtained from public databases, such as NCBI and Ensembl. For example, the amino acid sequence of human adenosine receptor A2A is registered in NCBI with the accession number NP_000666.2. For example, the amino acid sequence of mouse adenosine receptor A2A is also registered in NCBI with the accession number NP_033760.2. For example, the amino acid sequence of human adenosine receptor A2B is also registered in NCBI with the accession number NP_000667.1. For example, the amino acid sequence of mouse adenosine receptor A2B is also registered in NCBI with the accession number NP_031439.2.

Methods for reducing the activity of proteins such as adenosine receptors will be explained below.

The expression "activity of protein is reduced" means that the activity of the protein is decreased compared with that of an unmodified cell. The expression "activity of protein is reduced" specifically means that the per-cell activity of the protein is decreased compared with that of an unmodified cell. The term "unmodified cell" used here means a control cell that has not been modified so that activity of a target protein is reduced, and may specifically mean a cell that normally expresses the target protein that normally functions. Examples of such a control cell include the host cell exemplified above. That is, according to one embodiment, the activity of the protein may be reduced compared with CHO, such as CHO-DG44 and CHO-K1. In another embodiment, the activity of the protein may be reduced compared with HEK, such as HEK293 and HEK293T. In another embodiment, the activity of the protein may be reduced compared with COS, such as COS-1. The expression "activity of protein is reduced" may also mean that the activity of the protein has been completely lost. The expression "activity of protein is reduced" may more specifically mean that the number of molecules of the protein per cell is reduced and/or the function per molecule of the protein is reduced compared with an unmodified cell. That is, "activity" used in the expression "activity of protein is reduced" is not limited to the catalytic activity of the protein, but may also mean transcription amount (mRNA amount) or translation amount (protein amount) of the gene encoding the protein. The "number of molecules of the protein per cell" may mean average number of molecules of the protein per cell. The expression "number of molecules of the protein per cell is decreased" may also mean that the protein is not present at all. The expression "function of protein per molecule is reduced" may also mean that the function of each molecule of the protein has been completely lost. The degree of reduction in the activity of the protein is not particularly limited as long as the desired effect is achieved (e.g., in the case of adenosine receptors, as long as response of an adenosine receptor to a test substance can be reduced to a desired degree). The activity of the protein may be reduced to, for example, 10% or lower, 5% or lower, 2% or lower, 1% or lower, 0.5% or lower, 0.2% or lower, 0.1% or lower, or 0% of that of the unmodified cell.

Such a modification of a protein that the activity the protein is reduced can be achieved by, for example, reducing the expression of the gene encoding the protein. The expression "expression of a gene is reduced" means that the expression of the gene is reduced compared with that of an unmodified cell. The expression "expression of a gene is reduced" specifically means that the expression of the gene per cell is reduced compared with that of an unmodified cell. The expression "per-cell expression amount of a gene" may mean average per-cell expression amount of the gene. The expression "expression of a gene is reduced" may more specifically mean that transcription amount of the gene (mRNA amount) is reduced, and/or translation amount of the gene (protein amount) is reduced. The expression "expression of a gene is reduced" may also mean that the gene is not expressed at all. The expression "expression of a gene is reduced" is also expressed as "expression of a gene is attenuated". Expression of the gene may be reduced to, for example, 10% or less, 5% or less, 2% or less, 1% or less, 0.5% or less, 0.2% or less, 0.1% or less, or 0% of that of an unmodified cell.

The reduction of expression of a gene may be due to, for example, reduction in transcription efficiency, reduction in translation efficiency, or a combination thereof. Reduction of gene expression can be achieved by, for example, modifying an expression control sequence of the gene. The term "expression control sequence" is a generic term for sites that affect expression of a gene, such as a promoter. The expression control sequence can be determined by using, for example, promoter search vectors or gene analysis software such as GENETYX. When modifying an expression control sequence, the expression control sequence is preferably modified for one or more nucleotides, more preferably two or more nucleotides, especially preferably three or more nucleotides. Reduction of gene transcription efficiency can be achieved by, for example, replacing the promoter of a gene on the chromosome with a weaker promoter. The "weaker promoter" means a promoter that weakens transcription of the gene compared with the originally existing wild-type promoter. Examples of such a weaker promoter include inducible promoters. That is, inducible promoters can function as a weaker promoter under non-inducible conditions (e.g., in the absence of an inducer). In addition, a part or all of the region of the expression control sequence may be deleted (made deficient). The reduction of gene expression can also be achieved by, for example, introducing such a mutation that the expression of the gene is reduced into the coding region of the gene. For example, expression of a gene can be reduced by replacing a codon in the coding region of the gene with a synonymous codon that is used at a lower frequency in the host. Expression of a gene itself can also be reduced by, for example, disrupting the gene as described later. Reduction of gene expression can also be achieved by, for example, RNA interference.

Such a modification of a protein that the activity thereof is reduced can also be achieved by, for example, disrupting a gene that encodes the protein. The expression "gene is disrupted" means that the gene is altered so that it no longer produces a protein that can normally function. The expression "a protein that can normally function is not produced" means that no protein is produced from the gene, or a protein showing reduced per-molecule function (e.g., activity or property) or not showing such function is produced from the gene.

Gene disruption can be achieved by, for example, deleting (making deficient) the gene on a chromosome. The term "deletion of a gene" means deletion of a part or all of the coding region of the gene. In addition, the entire gene including the sequences before and after the coding region of the gene on the chromosome can be deleted. The sequences before and after the coding region of the gene may include, for example, gene expression control sequences. The region to be deleted may be any region, such as an N-terminal region (encoding the N-terminal side of the protein), internal region, or C-terminal region (encoding the C-terminal side of the protein), so long as reduction of protein activity can be achieved. Deletion of a longer region can usually more reliably inactivate the gene. The region to be deleted may be, for example, 10% or more, 20% or more, 30% or more, 40% or more, 50% or more, 60% or more, 70% or more, 80% or more, 90% or more, or 95% or more of the total length of the coding region of the gene. The sequences before and after the region to be deleted should not preferably have the same reading frames. Mismatch of reading frames can result in a frameshift downstream of the region to be deleted.

Gene disruption can also be achieved by, for example, introducing amino acid substitution (missense mutation), termination codon (nonsense mutation), or addition or deletion of one or two nucleotides (frameshift mutation) into the coding region of a gene on a chromosome (Journal of Biological Chemistry 272:8611-8617 (1997); Proceedings of the National Academy of Sciences, USA, 95 5511-5515 (1998); Journal of Biological Chemistry 26 116, 20833-20839 (1991)).

Gene disruption can also be achieved by, for example, inserting another nucleotide sequence into the coding region of the gene on the chromosome. The insertion site can be in any region of the gene, but the longer the sequence to be inserted, the more reliably the gene can be inactivated. Preferably, the sequences before and after the insertion site should not have the same reading frames. Mismatch of the reading frame can result in a frameshift downstream of the insertion site. The other nucleotide sequence is not particularly limited as long as it reduces or eliminates the activity of the encoded protein, but examples include, for example, marker genes such as antibiotic resistance genes and genes useful in production of a target substance.

Disruption of a gene may be performed, in particular, so that the amino acid sequence of the encoded protein is deleted (made deficient). In other words, such a modification that the activity of the protein is reduced can be achieved by, for example, deleting an amino acid sequence (a part or all of the region of the amino acid sequence) of the protein, specifically, by modifying the gene so as to encode a protein in which the amino acid sequence (a part or all of the region of the amino acid sequence) is deleted. The expression "an amino acid sequence of a protein is deleted" means that a part or all of the region of the amino acid sequence of the protein is deleted. In addition, "an amino acid sequence of a protein is deleted" also means that the original amino acid sequence is no longer present in the protein, and also means that the original amino acid sequence is changed to another amino acid sequence. In other words, for example, a region changed to another amino acid sequence by frameshift may be regarded as a deleted region. Deletion of an amino acid sequence in a protein typically shortens the total length of the protein, but there may be cases where the total length of the protein is unchanged or even extended. For example, a deletion of a part or all of the coding region of a gene can result in deletion of the region encoded by the deleted region in the amino acid sequence of the encoded protein. For example, by introducing a termination codon into the coding region of a gene, the region encoded by the region downstream of the introduced site can also be deleted in the amino acid sequence of the encoded protein. For example, by frameshift in the coding region of a gene, the region encoded by the frameshift site can also be deleted. For the position and length of the region to be deleted in the deletion of an amino acid sequence, the descriptions of the position and length of the region to be deleted in the deletion of a gene can be correspondingly applied.

The method for modifying chromosomes can be selected according to various conditions, such as type of the modification and type of cell. For example, site-specific modification of chromosomes in higher eukaryotic cells, such as animal cells, can be performed by techniques using various nucleases. Specific examples of such methods include CRISPR/Cas (CRISPR, clustered regularly interspaced short palindromic repeat; Cas, CRISPR-associated protein) method, TALEN (transcription-activator like effector nuclease) method, and ZFN (zinc finger nuclease) method. In these methods, Cas, TALEN, and ZFN are used as the nuclease, respectively. For example, a target site on a chromosome may be deleted by non-homologous end joining, or a target site on a chromosome may be replaced with another nucleotide sequence (such as a sequence containing a marker gene) by homologous recombination. For example, after a disrupted gene that has been modified so that it does not produce a normally functioning protein is created, a donor DNA containing the disrupted gene can be introduced into a host cell to cause homologous recombination between the disrupted gene and the wild-type gene on the chromosome and thereby replace the wild-type gene on the chromosome with the disrupted gene. The disrupted gene can be a gene in which a part or all of the coding region of the gene is deleted, a gene into which a missense mutation is introduced, a gene into which a nonsense mutation is introduced, a gene into which a frameshift mutation is introduced, or a gene into which an insertion sequence such as a transposon or marker gene is inserted.

Modifications that reduce the activity of the protein may be achieved by, for example, mutation treatments. Mutation treatments include X-ray irradiation, UV irradiation, and treatments with mutation agents such as N-methyl-N'-nitro-N-nitrosoguanidine (MNNG), ethyl methanesulfonate (EMS), and methyl methanesulfonate (MMS).

The above methods for reducing protein activity may be used individually, or in any combination.

The reduction of activity of a protein can be confirmed by measuring the activity of that protein. Activity of an adenosine receptor can be measured by, for example, measuring response of the adenosine receptor to adenosine. Response of an adenosine receptor to adenosine can be measured by, for example, contacting adenosine with cells and measuring activation of the adenosine receptor. Activation of an adenosine receptor can be measured in the same way as the measurement of activation of olfactory receptors.

The reduction of activity of a protein can also be confirmed by confirming reduction of expression of the gene encoding the protein. Reduction of gene expression can be confirmed by confirming reduction of transcription amount of the gene, or by confirming reduction of amount of the protein expressed from the gene.

Reduction of gene transcription amount can be confirmed by comparing amount of mRNA transcribed from the gene with that of an unmodified cell. mRNA amounts can be evaluated by Northern hybridization, RT-PCR, microarray method, RNA-Seq, and other methods (Sambrook, J., et al., Molecular Cloning: A Laboratory Manual/Third Edition, Cold Spring Harbor Laboratory Press, Cold Spring Harbor (USA), 2001). mRNA amount may be reduced to, for example, 10% or less, 5% or less, 2% or less, 1% or less, 0.5% or less, 0.2% or less, 0.1% or less, or 0% of that of an unmodified cell.

Reduction of protein amount can be confirmed by performing SDS-PAGE and checking intensities of the separated protein bands. Reduction of protein amount can also be confirmed by Western blotting using antibodies (Sambrook, J., et al., Molecular Cloning: A Laboratory Manual/Third Edition, Cold Spring Harbor Laboratory Press, Cold Spring Harbor (USA), 2001). The amount of protein (e.g., number of molecules per cell) may be reduced to, for example, 10% or less, 5% or less, 2% or less, 1% or less, 0.5% or less, 0.2% or less, 0.1% or less, or 0% of that of an unmodified cell.

Disruption of a gene can be confirmed by determining nucleotide sequence of a part or all of the gene, restriction enzyme map, full length, and so forth of the gene depending on the means used for the disruption.

The methods for reducing the activity of proteins described above can be used to reduce activity of any protein or expression of any gene.

A cell having an olfactory receptor gene may be used as it is, or may be used as a cell having an olfactory receptor after the olfactory receptor gene is appropriately expressed (a cell of the present invention). In other words, if the cell having an olfactory receptor gene already express the olfactory receptor gene, the cell may be used as it is as a cell having an olfactory receptor (cells of the present invention). By allowing a cell having an olfactory receptor gene to express the olfactory receptor gene, a cell having an olfactory receptor (a cell of the present invention) can also be obtained. For example, by culturing a cell having an olfactory receptor gene, the olfactory receptor gene can be expressed, and thereby a cell having an olfactory receptor (a cell of the present invention) can be obtained. Specifically, for example, after introduction (e.g. transfection) of an olfactory receptor gene, culture of the host cell can be continued to allow expression of the olfactory receptor gene. The medium composition and culture conditions are not particularly limited so long as the cell having the olfactory receptor gene can be maintained (e.g., proliferated) and the olfactory receptor gene is expressed. During the culture, a cell having the olfactory receptor gene may or may not proliferate. The medium composition and culture conditions can be appropriately determined according to various conditions, such as type of the host cell. For example, culture can be performed by using a usual culture medium and usual conditions used for culturing a cell such as an animal cell as it is, or with appropriate modifications. For example, specific examples of media that can be used for culturing animal cells include Opti-MEM medium (Thermo Fisher Scientific), DMEM medium, RPMI 1640 medium, and CD293 medium. The culture can be performed, for example, as static culture at 36 to 38°C in an atmosphere containing 5% CO₂ or the like. Selection agents and expression inducers can be used if necessary.

The expression of olfactory receptors can be confirmed by measuring response of olfactory receptors to substances that elicit the response of olfactory receptors. The expression of olfactory receptors can also be confirmed by measuring the amount of mRNA transcribed from the olfactory receptor gene or by detecting the olfactory receptors by Western blotting using antibodies.

The cell of the present invention may be used in the method of the present invention, for example, either as they are (as contained in the culture medium) or after recovered from the medium. The culture product and the cell recovered therefrom may also be used in the method of the present invention, for example, optionally after such treatments as washing, concentration, dilution, and immobilization. As described above, for example, the cell of the present invention may be used in the form of a cell isolated to a desired degree, or may be used in the form of a cell contained in any material such as culture product.

A cell membrane can also be prepared from the cell of the present invention and used in the method of the present invention. A cell membrane prepared from the cell of the present invention is a cell membrane having an olfactory receptor. Specifically, a cell membrane having an olfactory receptor can be obtained as, for example, a membrane fraction after the cell of the present invention is disrupted. Such a cell membrane having an olfactory receptor may be used, for example, as it is or after dispersed in an artificial lipid bilayer. Such a cell membrane having an olfactory receptor may also be used in the form of a vesicle (i.e., a vesicle prepared from the cell membrane).

The cell membrane can be used, for example, to create spaces demarcated by the cell membrane. The cell membrane can be used, for example, to demarcate two spaces, such as two wells. That is, the cell membrane can be used, for example, to provide a reaction system with two spaces, such as two wells, wherein the two spaces are demarcated from each other by the cell membrane. Such two spaces need only be demarcated by the cell membrane for at least a part of their boundary. Such a reaction system can be provided as, for example, an ion channel measurement device (Kawano R. et al., Automated Parallel Recordings of Topologically Identified Single Ion Channels, Scientific Reports, 3, No. 1995 (2013)).

### <3> Method of the present invention

The method of the present invention can be performed in vitro.

The step (A) is the step of contacting the olfactory receptor with a test substance. That is, the olfactory receptor can be first brought into contact with a test substance.

The system in which the contact between the olfactory receptor and the test substance is performed is also referred to as a "reaction system". The contact between the olfactory receptor and the test substance can be performed in an appropriate liquid. The liquid in which the contact between the olfactory receptor and the test substance is attained is also referred to as a "reaction liquid". That is, for example, the olfactory receptor and the test substance can be contacted by allowing the olfactory receptor and the test substance to coexist in an appropriate reaction liquid. Specifically, by, for example, dissolving, suspending, or dispersing the olfactory receptor (e.g., such a form as exemplified above, such as a cell having an olfactory receptor) and the test substance in an appropriate liquid medium, the olfactory receptor and the test substance can be contacted. Examples of the liquid medium include aqueous media such as water or aqueous buffer. When two or more kinds of ingredients are contacted with the olfactory receptor, the contacts between them and the olfactory receptor may or may not be simultaneously started. That is, for example, after starting contact of one kind of ingredient with the olfactory receptor, another kind of ingredient may be added to the reaction system. The reaction conditions (conditions under which the contact between the olfactory receptor and the test substance is attained) are not particularly limited, so long as the response of the olfactory receptor to the test substance can be measured. The reaction conditions can be appropriately set according to various conditions such as form of the olfactory receptor at the time of use, type of test substance, and method used to measure the response of the olfactory receptor. As the reaction conditions, for example, known reaction conditions for measuring interactions between substances, such as interactions between proteins and ligands, may be used as they are, or with appropriate modification. The concentration of the test substance may be, for example, 0.01% (w/w) or higher, 0.02% (w/w) or higher, 0.05% (w/w) or higher, 0.1% (w/w) or higher, 0.2% (w/w) or higher, 0.5% (w/w) or higher, or 1% (w/w) or higher, and 15% (w/w) or lower, 10% (w/w) or lower, 7% (w/w) or lower, 5% (w/w) or lower, 2% (w/w) or lower, 1% (w/w) or lower, 0.5% (w/w) or lower, 0.2% (w/w) or lower, or 0.1% (w/w) or lower, or in a range defined with any consistent combination of the above lowest and highest concentrations. The concentration of the test substance may be, specifically, for example, 0.01 to 15% (w/w), 0.05 to 7% (w/w), or 0.1 to 5% (w/w). The concentration of the test substance may be, for example, 0.01 nM to 500 mM, 10 nM to 100 mM, 1 µM to 10 mM, or 3 µM to 1 mM. The concentration of the olfactory receptor may be, for example, 1 pg/mL to 10 mg/mL. When cells having olfactory receptors are used, concentration of the cells having olfactory receptors may be, for example, 10 cells/mL to 10,000,000 cells/mL. The contact between the olfactory receptor and the test substance may or may not be terminated at an appropriate time point. The contact between the olfactory receptor and the test substance may normally be continued until the time of measuring response of the olfactory receptor to the test substance. The duration of the contact between the olfactory receptor and the test substance may be, for example, 0.1 second or longer, 0.5 seconds or longer, 1 second or longer, 5 seconds or longer, 10 seconds or longer, 30 seconds or longer, 1 minute or longer, 5 minutes or longer, 10 minutes or longer, 30 minutes or longer, 1 hour or longer, or 2 hours or longer, and 24 hours or shorter, 12 hours or shorter, 6 hours or shorter, 2 hours or shorter, or 1 hour or shorter, or in a range defined with any consistent combination of the above shortest and longest durations. The duration of the contact between the olfactory receptor and the test substance may be specifically, for example, from 1 to 6 hours. The reaction system may contain other ingredients in addition to the olfactory receptor (e.g., such a form as exemplified above, such as a cell having an olfactory receptor) and the test substance, so long as the response of the olfactory receptor to the test substance can be measured. The other ingredients can be chosen according to various conditions, such as form of the olfactory receptor at the time of use, type of the test substance, and method used to measure response of the olfactory receptor. Examples of the other ingredients include salts such as calcium salts, carbon sources such as glucose, other medium ingredients, and pH buffers.

When inactivation of an olfactory receptor by a test substance is measured, the contact between the olfactory receptor and the test substance can be performed, for example, in the presence of a substance that activates the olfactory receptor (hereinafter also called "olfactory receptor activating substance"). The expressions "contact an olfactory receptor and a test substance in the presence of an olfactory receptor activating substance", "contact an olfactory receptor and an olfactory receptor activating substance in the presence of a test substance", and "contact an olfactory receptor with an olfactory receptor activating substance and a test substance" may be used interchangeably and synonymously. The descriptions for the contact between the olfactory receptor and the test substance can be correspondingly applied to the contact between the olfactory receptor and the olfactory receptor activating substance. The contact of the olfactory receptor activating substance and the test substance with the olfactory receptor may or may not be started simultaneously. For example, the test substance and the olfactory receptor activating substance may be mixed beforehand, and then contacted with the olfactory receptor. Alternatively, for example, the olfactory receptor activating substance may be further added to the reaction system in which the contact between the test substance and the olfactory receptor has already been started, or the test substance may be further added to the reaction system in which the contact between the olfactory receptor activating substance and the olfactory receptor has already been started. Concentration of the olfactory receptor activating substance may be, for example, 0.01 nM to 500 mM, 10 nM to 100 mM, 1 µM to 10 mM, or 3 µM to 1 mM. When the contact between the olfactory receptor and the test substance is attained in the presence of the olfactory receptor activating substance, "duration of the contact between the olfactory receptor and the test substance" may be read as "duration of the contact of the olfactory receptor with the olfactory receptor activating substance and the test substance".

The olfactory receptor activating substance is not particularly limited so long as it can activate the olfactory receptor. The olfactory receptor activating substance may consist of a single ingredient (i.e., pure substance) or a combination of two or more kinds of ingredients (i.e., mixture). If the olfactory receptor activating substance consists of a mixture, the number of types and composition ratios of the ingredients constituting the mixture are not particularly limited. The olfactory receptor activating substance may be a known substance or novel substance. The olfactory receptor activating substance may be a natural or artificial substance. The olfactory receptor activating substance may or may not be known to be able to activate the olfactory receptor. For example, a substance that can activate an olfactory receptor may be selected from such test substances as exemplified above and used as the olfactory receptor activating substance.

The step (A) is performed under the following condition (X):
(X) a condition that the response of the adenosine receptor to the test substance is reduced.

The above condition (X) may be achieved because, for example, the cell of the present invention has the following characteristic (X1) or (X2):
(X1) the cell is modified so that the activity of the adenosine receptor is reduced; or
(X2) the cell does not inherently have the adenosine receptor.

The characteristics (X1) and (X2) are as described above.

The condition (X) may be achieved by, for example, performing the step (A) in the presence of an adenosine receptor antagonist. When the condition (X) is achieved by performing the step (A) in the presence of an adenosine receptor antagonist, and inactivation of the olfactory receptor induced by the test substance is measured, the olfactory receptor and the olfactory receptor activating substance are contacted also in the presence of an adenosine receptor antagonist.

As the adenosine receptor antagonist, antagonists of selected adenosine receptors can be used. For example, examples of adenosine receptor A2A antagonist include caffeine, theophylline, istradefylline, CGS-15943, SCH-58261, SCH-442416, and ZM-241385. As the adenosine receptor A2A antagonist, in particular, CGS-15943 can be mentioned. Examples of antagonist of adenosine receptor A2B include, for example, caffeine, theophylline, CGS-15943, CVT-6883, MRS-1706, MRS-1754, MRE-2029-F20, PSB-603, PSB-0788, and PSB-1115. As the antagonist of adenosine receptor A2B, in particular, CGS-15943 can be mentioned.

The concentration of the adenosine receptor antagonist is not particularly limited so long as it can reduce the response of the adenosine receptor to the test substance to a desired degree. The response of the adenosine receptor to the test substance may be reduced to, for example, 10% or less, 5% or less, 2% or less, 1% or less, 0.5% or less, 0.2% or less, 0.1% or less, or 0% of that obtained in the absence of adenosine receptor antagonist. Concentration of the adenosine receptor antagonist can be appropriately set according to various conditions, for example, type of the adenosine receptor antagonist. The concentration of the adenosine receptor antagonist may be, for example, 0.05 µM or higher, 0.1 µM or higher, 0.2 µM or higher, 0.5 µM or higher, or 1 µM or higher, and 1000 µM or lower, 100 µM or lower, 10 µM or lower, or 5 µM or lower, or may be in a range defined by any combination of the above highest and lowest concentrations. The concentration of the adenosine receptor antagonist may be, for example, 0.05 to 1000 µM, 0.1 to 100 µM, or 0.2 to 10 µM.

The time of supplying the adenosine receptor antagonist to the reaction system is not particularly limited, so long as it can reduce the response of the adenosine receptor to the test substance to a desired degree. The adenosine receptor antagonist may be supplied to the reaction system, for example, before or simultaneously with the supply of the test substance. When an olfactory receptor activating substance is used, the adenosine receptor antagonist may be supplied to the reaction system, for example, before or simultaneously with the supply of the olfactory receptor activating substance.

The step (B) is the step of measuring the response of the olfactory receptor to the test substance. That is, the response of the olfactory receptor to the test substance can then be measured. The expression that the olfactory receptor responds to a substance is also referred to as "the substance elicits a response of the olfactory receptor".

Examples of the response of the olfactory receptor to the test substance include activation or inactivation of the olfactory receptor by the test substance.

The time for measuring the response of the olfactory receptor to the test substance is not particularly limited so long as the response of the olfactory receptor to the test substance is elicited in such a degree that the response can be measured, when the test substance is a substance that elicits a response of the olfactory receptor. The time of the measurement of the olfactory receptor response to the test substance can be appropriately determined according to various conditions, such as form of the olfactory receptor at the time of use, type of the test substance, and method for measuring the olfactory receptor response. Specifically, the time of measuring the response of the olfactory receptor to the test substance can be any appropriate time point from the time point at which contact between the olfactory receptor and the test substance has been started to the time point at which the response of the olfactory receptor to the test substance disappears. The time of measuring the response of the olfactory receptor to the test substance may be, for example, the time point at which the maximum response of the olfactory receptor to the test substance is obtained (e.g., when the degree of activation or inactivation of the olfactory receptor induced by the test substance is maximized). The time of measuring the olfactory receptor response to the test substance may be, for example, after 0.1 second, 0.5 seconds, 1 second, 5 seconds, 10 seconds, 30 seconds, 1 minute, 5 minutes, 10 minutes, 30 minutes, 1 hour, or 2 hours from the start of the contact between the olfactory receptor and the test substance, and up to 24 hours, 12 hours, 6 hours, 2 hours, or 1 hour from the start of the contact between the olfactory receptor and the test substance, or in a range defined by any consistent combination of the above earliest and latest time points. The time of measuring the response of the olfactory receptor to the test substance may be, specifically, for example, a time after 1 to 6 hours from the time point at which the contact between the olfactory receptor and the test substance has been started. If the contact between the olfactory receptor and the test substance is performed in the presence of an olfactory receptor activating substance, "the time point at which the contact between the olfactory receptor and the test substance has been started" may be read as "the time point at which the contact of the olfactory receptor with the olfactory receptor activating substance and the test substance has been started".

The activation or inactivation of the olfactory receptor induced by the test substance may be determined by using the degree of activation of the olfactory receptor (degree of activation D1) observed when the aforementioned step (A) is performed (i.e., under the condition for the contact between the olfactory receptor and the test substance) as an indicator. In other words, the step (B) may be, for example, (B1) the step of measuring the degree of activation D1.

The activation or inactivation of the olfactory receptor induced by the test substance may be determined by, specifically, comparing degree of the activation of the olfactory receptor (degree of activation D1) observed when the above step (A) is performed (i.e., under the condition of the contact between the olfactory receptor and the test substance) with degree of the activation of the olfactory receptor observed under control condition (degree of activation D2).

The "control condition" refers to the following condition (C2-1) or (C2-2):
(C2-1) a condition that the olfactory receptor is not contacted with the test substance;
(C2-2) a condition that the olfactory receptor is contacted with the test substance, where the concentration of the test substance is lower than the concentration of the test substance used in the step (A).

If the olfactory receptor and the test substance are contacted in the presence of the olfactory receptor activating substance, the above conditions (C2-1) and (C2-2) can be read as follows:
(C2-1) a condition that the olfactory receptor and the olfactory receptor activating substance are contacted, but the olfactory receptor and the test substance are not contacted (i.e., a condition that the olfactory receptor and the olfactory receptor activating substance are contacted in the absence of the test substance);
(C2-2) a condition that the olfactory receptor and the test substance are contacted in the presence of the olfactory receptor activating substance, where the concentration of the test substance is lower than the concentration of the test substance used in the step (A).

In other words, the activation or inactivation of the olfactory receptor induced by the test substance may be determined by, for example, using the difference in the degree of activation of the olfactory receptor resulting from difference of the presence or absence of the test substance or difference in concentration of the test substance as an indicator.

Examples of the above condition (C2-1) include a condition attained before the olfactory receptor is contacted with the test substance. Examples of the above condition (C2-1) also includes a condition attained after the olfactory receptor has been contacted with the test substance, where the test substance has been substantially (e.g., completely) removed from the reaction system and the response of the olfactory receptor to the test substance has substantially (e.g., completely) disappeared. The concentration of the test substance used in the above condition (C2-2) is not particularly limited so long as the concentration is such a concentration that a measurable difference is observed between the degree of activation D1 and the degree of activation D2. The concentration of the test substance in the above condition (C2-2) may be, for example, 90% or lower, 70% or lower, 50% or lower, 30% or lower, 20% or lower, 10% or lower, 5% or lower, or 1% or lower of the concentration of the test substance used in the above step (A). The control condition is not particularly limited except for the presence or absence or concentration of the test substance, so long as the olfactory receptor response to the test substance can be evaluated. The control condition may be, for example, the same as the condition of the above step (A), except for the presence or absence or concentration of the test substance. For example, when the above condition (X) is achieved by performing the step (A) in the presence of an adenosine receptor antagonist, the contact between the olfactory receptor and a substance in the control condition is also performed in the presence of an adenosine receptor antagonist.

The method of the present invention may include the step of measuring the degree of activation D2. The degree of activation D1 and the degree of activation D2 may be measured in a single reaction system with a time lag, or they may be measured in separate reaction systems simultaneously or with a time lag. The degree of activation D2 may be measured before or after measuring the degree of activation D1. For example, after measuring the degree of activation D2, the test substance may be added to the reaction system and the degree of activation D1 may be measured.

When the degree of activation D1 is high, it may be judged that activation of the olfactory receptor by the test substance is observed. Specifically, when the degree of activation D1 is higher than the degree of activation D2, it may be judged that activation of the olfactory receptor by the test substance is observed. For example, if the ratio of the degree of activation D1 to the degree of activation D2 (i.e., D1/D2) is 1.5 or higher, 2 or higher, 3 or higher, 5 or higher, 10 or higher, 20 or higher, 50 or higher, or 100 or higher, it may be determined that activation of the olfactory receptor by the test substance is observed. Examples of the ratio of the degree of activation D1 to the degree of activation D2 include the normalized response values described in the examples.

When the degree of activation D1 is low, it may be judged that inactivation of the olfactory receptor by the test substance has been observed. Specifically, when the degree of activation D1 is lower than the degree of activation D2, it may be judged that inactivation of the olfactory receptor by the test substance has been observed. For example, if the ratio of the degree of activation D1 to the degree of activation D2 (i.e., D1/D2) obtained in the presence of an olfactory receptor activating substance is 0.8 or lower, 0.7 or lower, 0.6 or lower, 0.5 or lower, 0.4 or lower, 0.3 or lower, 0.2 or lower, or 0.1 or lower, it may be judged that inactivation of the olfactory receptor by the test substance has been observed. Examples of the ratio of the degree of activation D1 to the degree of activation D2 obtained in the presence of the olfactory receptor activating substance include the normalized response values obtained in the presence of the olfactory receptor activating substance described in the examples.

The method for measuring response of the olfactory receptor to the test substance is not particularly limited. The method for measuring response of the olfactory receptor to the test substance can be selected according to various conditions, such as form of the olfactory receptor at the time of use and type of response to be measured. That is, response of the olfactory receptor to the test substance can be measured by, for example, an appropriate method with which activation or inactivation of the olfactory receptor induced by the test substance can be measured.

The method for measuring activation or inactivation of the olfactory receptor induced by the test substance is not particularly limited. Activation or inactivation of the olfactory receptor induced by the test substance can be measured by, for example, known methods for measuring activity of receptors such as olfactory receptors. Examples of such methods include, for example, methods for measuring intracellular calcium concentration and methods for measuring intracellular cAMP concentration. In other words, activation or inactivation of the olfactory receptor by the test substance can be measured by, for example, using intracellular calcium concentration or intracellular cAMP concentration as an indicator. In other words, activation or inactivation of the olfactory receptor induced by the test substance can be specifically measured by, for example, measuring intracellular calcium concentration or intracellular cAMP concentration of cells having the olfactory receptor as an indicator. Activation or inactivation of the olfactory receptor induced by the test substance can be measured by, in particular, using intracellular cAMP concentration as an indicator. For example, it is known that, in HEK293T cells, if olfactory receptors are activated by aroma ingredients, they couple with intracellular G proteins (specifically, Gs such as Golf) to activate adenylate cyclase, thereby increasing the amount of intracellular cAMP amount (Kajiya K. et al., Molecular bases of odor discrimination: Reconstitution of olfactory receptors that recognize overlapping sets of odorants, Journal of Neuroscience, 2001, 21:6018-6025). Examples of the method for measuring intracellular cAMP concentration include, for example, ELISA and reporter assay. As the reporter assay, luciferase assay can be mentioned. By the reporter assay, intracellular cAMP concentration can be measured by using a reporter gene (such as luciferase genes) configured to be expressed in a cAMP concentration-dependent manner. As the method for measuring intracellular calcium concentration, for example, calcium imaging can be mentioned. By the calcium imaging, intracellular calcium concentration can be measured by using a calcium indicator. Examples of the calcium indicator include calcium-sensitive fluorescent dyes and calcium-sensitive fluorescent proteins. Examples of the calcium-sensitive fluorescent dyes include, for example, Fura 2 and Fluo 4. Examples of the calcium-sensitive fluorescent proteins include, for example, Cameleon, TN-XL, GCaMP, and G-GECO. The term "calcium concentration" may mean concentration of free calcium ions.

The descriptions for the measurement of activation or inactivation of the olfactory receptor using a cell having the olfactory receptor can be correspondingly applied to the cases using a cell membrane having the olfactory receptor. As an example of the cases using a cell membrane having the olfactory receptor, in particular, the case where a cell membrane having the olfactory receptor is used in the form of cell membrane having an internal space.

For example, by using a cell membrane having the olfactory receptor so that spaces demarcated by the cell membrane are generated, activation or inactivation of the olfactory receptor can be measured in the same manner as that used when a cell having the olfactory receptor is used. Specifically, for example, if a cell membrane is used so as to demarcate two spaces, i.e., if the cell membrane is used to provide a reaction system with two spaces, where the two spaces are demarcated from each other by the cell membrane, activation or inactivation of the olfactory receptor can be measured in the same manner as that used when a cell having the olfactory receptor is used. In such a case, the space demarcated by the cell membrane can be considered the interior of the cell (also called "internal space"). Specifically, one of the two spaces can be regarded as the interior of the cell (also called "internal space") and the other as the exterior of the cell (also called "external space"). Of those spaces, the one containing the test substance can be considered the external space. In such a case, "intracellular calcium concentration" and "intracellular cAMP concentration" mentioned in the descriptions for the measurement of activation or inactivation of the olfactory receptor using a cell having the olfactory receptor can be read as "calcium concentration in the internal space" and "cAMP concentration in the internal space", respectively.

In any cases, measurable parameters can be selected according to the manner in which the olfactory receptor is used.

For "measuring a certain parameter and using it as an indicator to measure response of the olfactory receptor to the test substance", it is sufficient to obtain and use data reflecting the parameter so long as the response can be measured (i.e., it is possible to determine whether the response is recognized), and it is not necessary to obtain the value of the parameter itself. That is, when data reflecting the parameter are acquired, it is not necessary to calculate the value of the parameter itself from the data. Specifically, for example, when intracellular cAMP concentration is measured by a luciferase assay and used as an indicator to measure activation or inactivation of an olfactory receptor by a test substance, so long as such activation or inactivation can be measured (i.e., whether such activation or inactivation is observed can be determined), it is sufficient to obtain and use data reflecting the intracellular cAMP concentration (e.g., luminescence intensity), and it is not necessary to calculate the intracellular cAMP concentration itself from the data.

The purpose of measuring response of the olfactory receptor to the test substance is not particularly limited. The results of the measurement of response of the olfactory receptor to the test substance can be used for, for example, evaluating properties of the test substance (e.g., properties that can be associated with olfactory receptors, such as an ability to produce or suppress certain aroma). For example, if the selected olfactory receptor is an olfactory receptor that is activated by a substance that presents a specific aroma, it may be evaluated that the test substance is capable of presenting that aroma when the olfactory receptor is activated by the test substance, or that the test substance is capable of suppressing that odor when the olfactory receptor is inactivated by the test substance.

### Examples

Hereafter, the present invention will be more specifically explained with reference to nonlimiting examples.

### <1> Preparation of human olfactory receptor-expressing cells

### <1-1> Preparation of expression vectors for human olfactory receptors

As olfactory receptor, 352 kinds of human olfactory receptors (OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K13, OR4K14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5AU1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, OR5R1, OR5T1, OR5T2, OR5T3, OR5V1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4) were adopted.

The 352 kinds of human olfactory receptor genes were purchased from the TrueClone cDNA Clone Collection (OriGene). The fragments for subcloning of the 352 kinds of human olfactory receptor genes were each amplified by PCR using primers designed on the basis of sequence information registered at GenBank with the purchased human olfactory receptor genes as templates. Each of the amplified fragments for subcloning was subcloned into the Rho-pME18S vector (K. Kajiya et al., Journal of Neuroscience, 15 August, 2001, 21 (16) 6018-6025) downstream of the Rho tag sequence using the EcoRI and XhoI sites to obtain 352 kinds of human olfactory receptor expression vectors.

### <1-2> Preparation of olfactory receptor-expressing cells

HEK293T cells expressing each of the 352 kinds of olfactory receptors were prepared according to the following procedure. The gene mixture shown in Table 1 and the transfection reagent mixture shown in Table 2 were prepared and left stand at room temperature for 5 minutes. pcDNA3.1-microbat RTP1s is an expression vector for bat RTP1s, pcDNA3.1-Golf is an expression vector for human Golf, and pcDNA3.1-Ric8B is an expression vector for rat Ric8B (Japanese Patent Publication (Kokai) No. 2019-037197). The gene mixture and transfection reagent mixture were mixed, dispensed into each well of a poly-D-lysine-coated 384-well plate in a volume of 12.5 µL, and allowed to stand for 15 minutes in a clean bench. The HEK293T cells inoculated in 10-cm petri dishes on the previous day (2.5 × 10⁶ cells/10 cm petri dish) were prepared at a density of 1.2 × 10⁵ cells/mL, inoculated into each well of the 384-well plate in a volume of 25 µL, and cultured overnight in an incubator maintained at 37°C and 5% CO₂. In this way, there were obtained 352 kinds of cultures of HEK293T cells in which the expression vectors shown in Table 1 were transfected and which appropriately express the genes encoded by those expression vectors. As a control, the same procedure was performed by using the empty Rho-pME18S vector instead of the expression vector for the human olfactory receptor in the gene mixture shown in Table 1 to obtain culture of HEK293T cells transfected with the empty Rho-pME18S vector (hereinafter also called "control cells").

### [Table 1]

**Table 1**

| | |
|---|---|
| OPTI-MEM (GIBCO) | 6.2 µL |
| The expression vector for the human olfactory receptor | 0.0125 µg |
| pGL4.29[luc2P/CRE/Hygro] Vector, Promega | 0.0025 µg |
| pGL4.74[hRluc/TK] Vector, Promega | 0.00125 µg |
| pcDNA3.1-microbat RTP1s | 0.00250 µg |
| pcDNA3.1-Golf | 0.00125 µg |
| pcDNA3.1-Ric8B | 0.00125 µg |

### [Table 2]

**Table 2**

| | |
|---|---|
| OPTI-MEM (GIBCO) | 6.2 µL |
| Lipofection 2000 (Invitrogen) | 0.0425 µL |

### <2> Measurement of responses of olfactory receptors to test substances

### <2-1> Luciferase assay

The olfactory receptor-expressing cells were used to measure responses of the olfactory receptors to test substances. The test substances used were Japanese dashi (product name, Hondashi^{(R)} Irikodashi), soy sauce, mentsuyu (soup or dipping sauce for noodles), chicken broth (product name, Marudori Gara Soup^{(R)}), oyster sauce, beef extract, powdered coffee, and corn soup (product name, Knorr(R) Tsubu Tappuri Corn Cream).

The 352 olfactory receptors expressed in HEK293T cells couple with Golf to activate adenylate cyclase, thereby increasing the amount of intracellular cAMP. In this example, responses of the olfactory receptors to the test substances were measured by using a luciferase reporter gene assay, in which increase in intracellular cAMP amount is monitored as an increase in firefly luciferase-derived luminescence. The "luciferase reporter gene assay" is also referred to as "luciferase assay". The firefly luciferase is expressed from a firefly luciferase gene carried by pGL4.29[luc2P/CRE/Hygro] vector in an intracellular cAMP level-dependent manner. In addition, luminescence value derived from Renilla luciferase was used as an internal standard to correct errors due to differences of gene transfer efficiency and cell number among the wells. The Renilla luciferase is constitutively expressed from the Renilla luciferase gene carried by the pGL4.74[hRluc/TK] vector under the control of the TK promoter.

The media were removed from the cultures obtained in the section <1-2> mentioned above, and 60 µL of a test substance solution was added to each to obtain reaction solutions. Test substance solutions were each prepared by dissolving each test substance in CD293 (Life Technologies, Inc.). When an adenosine receptor antagonist (see below) was used, the adenosine receptor antagonist was also dissolved in each test substance solution. The reaction solution was placed in an incubator maintained at 37°C and 5% CO₂, and the cells were cultured for 4 hours to allow sufficient expression of the firefly luciferase gene in the cells. The luminescence value derived from firefly luciferase in the cells was measured and designated as "Luc value". The luminescence value derived from Renilla luciferase in the cells was also measured and designated as "hRLuc value". The luminescence value derived from each luciferase was measured by using Dual-Glo^{™} Luciferase Assay System (Promega) according to the product operation manual.

### <2-2> Calculation of olfactory receptor activity

The luminescence value derived from firefly luciferase induced by the test substance stimulation (Luc value) was divided by the luminescence value derived from Renilla luciferase in the same well (hRluc value) to obtain "Luc/hRluc value". The Luc/hRluc value of cells stimulated with the test substance was divided by the Luc/hRluc value of cells not stimulated with the test substance to obtain "fold increase". In addition, the fold increase of cells transfected with the olfactory receptor expression vector was divided by the fold increase of control cells (cells transfected with the empty vector Rho-pME18S) to obtain "normalized response". The common logarithm of the normalized response was used as the "olfactory receptor activity," a quantitative index of the intensity of the olfactory receptor response to the test substance.

### <3> Cellular response to adenosine and suppression of response by adenosine receptor antagonist

The luciferase assay was performed for control cells (cells transfected with the empty vector Rho-pME18S) according to the same procedures as those of <2-1> and <2-2> except that adenosine was used instead of the test substance, and the Luc/hRluc value of cells stimulated with adenosine was calculated. Separately, Luc/hRluc value of cells stimulated with 20 µM adenosine in the presence of an adenosine receptor antagonist was calculated. As the adenosine receptor antagonist, CGS-15943, an antagonist of adenosine receptors A2A and A2B, was used.

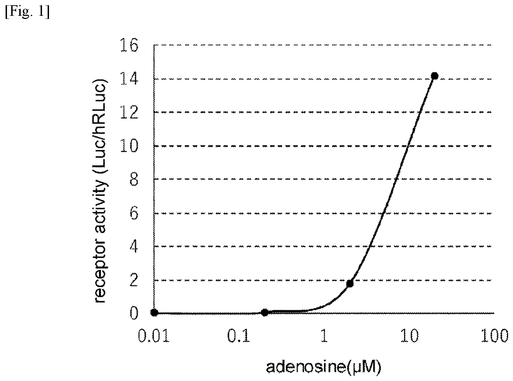

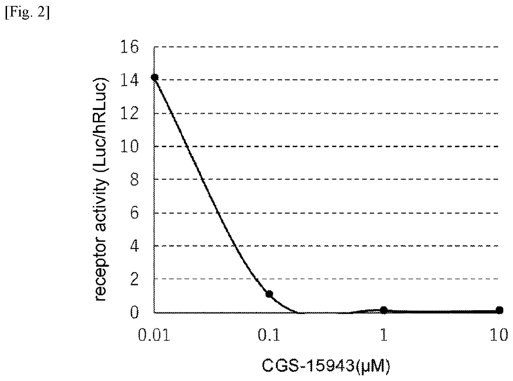

The results are shown in Figs. 1 and 2. The control cells responded to adenosine (Fig. 1), i.e., adenosine caused increase in cAMP in the control cells. The response to adenosine (i.e., adenosine-induced increase in cAMP) was inhibited by the adenosine receptor antagonist (Fig. 2).

### <4> Nonspecific cellular response to test substance and suppression of nonspecific response by adenosine receptor antagonist

The nonspecific responses of cells to a test substance (i.e., responses other than olfactory receptor responses to the test substance) were evaluated on the basis of the Luc/hRluc values of control cells (cells transfected with empty vector Rho-pME18S) and olfactory receptor-expressing cells obtained with test substance stimulation performed in the presence or absence of 10 µM adenosine receptor antagonist, CGS-15943.

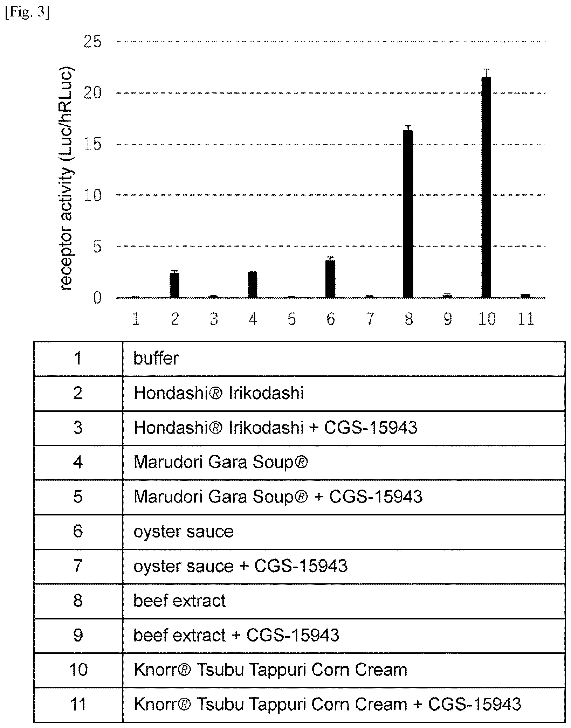

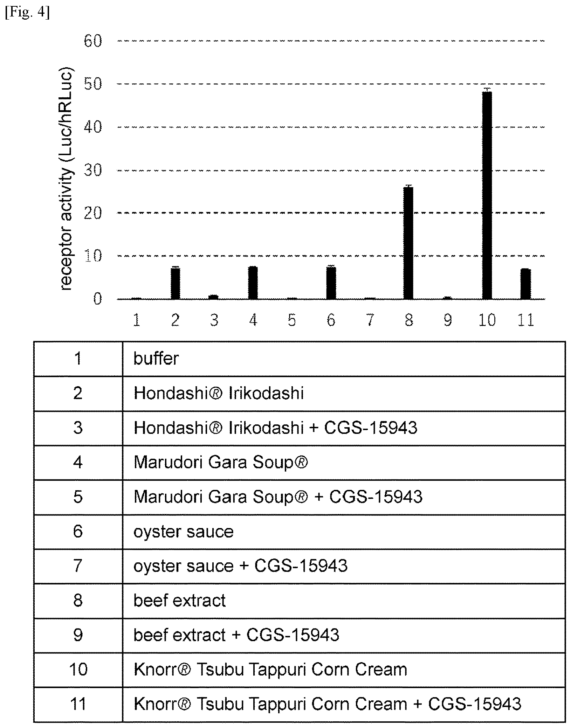

The results for the control cells are shown in Fig. 3. The control cells showed responses to several test substances, but the responses were almost completely suppressed by the adenosine receptor antagonist. The results for cells expressing olfactory receptor OR10G7 that respond to eugenol, as an example of olfactory receptor-expressing cells, are shown in Fig. 4. The OR10G7-expressing cells responded to several test substances, but these responses were partially or almost completely suppressed by the adenosine receptor antagonist. These results indicate that adenosine receptors (specifically, adenosine receptors A2A and/or A2B) are responsive to test substances and can interfere with the measurement of olfactory receptor responses to test substances, and that such adenosine receptor responses (i.e., nonspecific responses) can be suppressed by an adenosine receptor antagonist.

### <5> Correlation between adenosine concentration in test substance and nonspecific response of cells to test substance

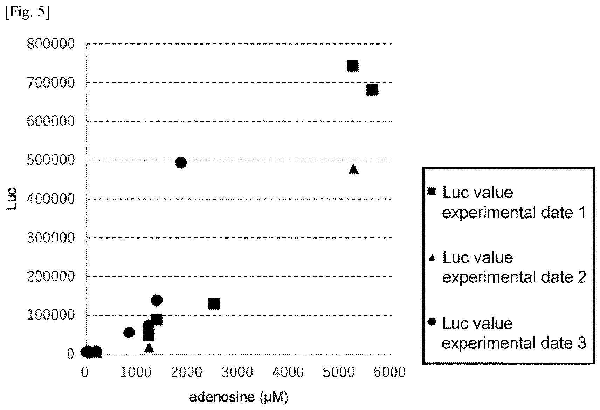

The adenosine concentrations in the test substances are shown in Table 3. The correlation between the concentration of adenosine and the Luc value of olfactory receptor OR10G7-expressing cells at the time of olfactory receptor activity measurement is shown in Fig. 5. The higher the adenosine concentration during the olfactory receptor activity measurement, the higher the Luc value, i.e., OR10G7-expressing cells showed a stronger response. This suggests that the concentration of adenosine in the test substances (specifically, the concentration of adenosine at the time of olfactory receptor activity measurement) and the nonspecific response of the cells to the test substances are positively correlated.

### [Table 3]

**Table 3**

| Test substance | Adenosine concentrations (nM) in 1% test substance solution | Concentrations of test substance solution (%) at the time of olfactory receptor activity measurement | Adenosine concentrations (nM) at the time of olfactory receptor activity measurement | Luc value experimental date 1 | Luc value experimental date 2 | Luc value experimental date 3 |
|---|---|---|---|---|---|---|
| Hondashi^{®} Irikodashi | 820 | 1.5 | 1230.2 | 47777 | 16996 | 72227 |
| soy sauce | 40.5 | 1.5 | 60.7 | | | 1553 |
| soy sauce | 40.5 | 0.30 | 12.1 | | | 2210 |
| mentsuyu | 45.4 | 1.0 | 45.4 | | | 5336 |
| Marudori Gara Soup^{®} | 1392 | 1.0 | 1391.8 | 85543 | | 137881 |
| oyster sauce | 841 | 3.0 | 2524.5 | 127892 | | |
| oyster sauce | 841 | 1.0 | 841.5 | | | 54043 |
| beef extract | 18783 | 0.30 | 5634.8 | 679155 | | |
| beef extract | 18783 | 0.10 | 1878.3 | | | 493582 |
| powdered coffee | 2073 | 0.10 | 207.3 | | 2098 | 4936 |
| Knorr^{®} Tsubu Tappuri Corn Cream | 1752 | 3.0 | 5255.3 | 742325 | 477386 | |

### <6> Improvement of detection sensitivity for olfactory receptor responses to test substances by adenosine receptor antagonist

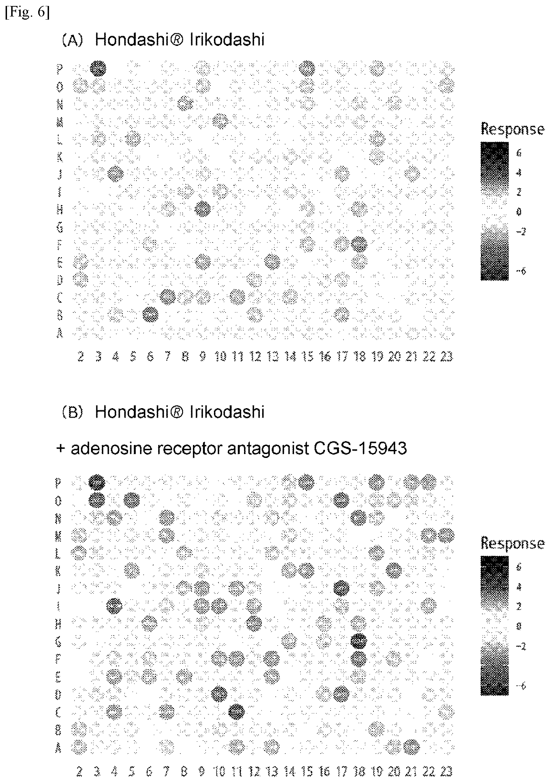

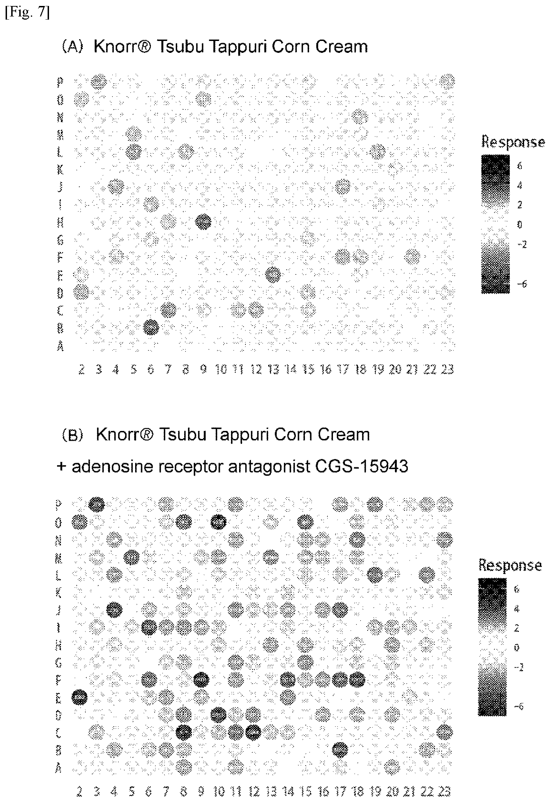

Olfactory receptor activities (common logarithms of normalized responses) measured by using Hondashi^{(R)} Irikodashi (dissolved in DMSO at 1.5%) as a test substance in the presence or absence of 20 µM adenosine receptor antagonist CGS-15943 are shown in Fig. 6. The olfactory receptor activities (common logarithms of normalized responses) measured by using Knorr(R) Tsubu Tappuri Corn Cream (dissolved in DMSO at 3%) as a test substance in the presence or absence of 20 µM adenosine receptor antagonist CGS-15943 are shown in Fig. 7. For both test substances, the use of the adenosine receptor antagonist increased the number of olfactory receptors that showed strong responses (activation or inactivation) to the test substances. That is, it was found that an adenosine receptor antagonist suppresses nonspecific responses of cells to test substances and increases detection sensitivity for the responses of olfactory receptors to test substances (i.e., activation or inactivation).

### Industrial applicability

According to the present invention, responses of olfactory receptors to a substance can be specifically measured.

## Claims

1. A method for measuring a response of an olfactory receptor to a test substance comprising the following steps (A) and (B):
(A) a step of contacting the olfactory receptor with the test substance; and
(B) a step of measuring the response of the olfactory receptor to the test substance, wherein the olfactory receptor is used in the form of being carried on a cell or on a cell membrane prepared from the cell, and
wherein the step (A) is performed under the following condition (X):
(X) a condition that a response of an adenosine receptor to the test substance is reduced.

2. The method according to claim 1, wherein the adenosine receptor is adenosine receptor A2A and/or A2B.

3. The method according to claim 1 or 2, wherein the condition (X) is achieved by performing the step (A) in the presence of an adenosine receptor antagonist.

4. The method according to claim 3, wherein the adenosine receptor antagonist is selected from the group consisting of caffeine, theophylline, istradefylline, CGS-15943, SCH-58261, SCH-442416, ZM-241385, CVT-6883, MRS-1706, MRS-1754, MRE-2029-F20, PSB-603, PSB-0788, and PSB-1115.

5. The method according to claim 3 or 4, wherein the adenosine receptor antagonist is used at a concentration of 0.05 to 1000 µM.

6. The method according to claim 1, wherein the condition (X) is achieved because the cell have the following characteristic (X1) or (X2):
(X1) the cell has been modified so that the activity of the adenosine receptor is reduced; or
(X2) the cell does not inherently have the adenosine receptor.

7. The method according to claim 6, wherein the cell have been modified so as not to have the adenosine receptor.

8. The method according to any one of claims 1 to 7, wherein the response of the olfactory receptor is activation of the olfactory receptor.

9. The method according to any one of claims 1 to 7, wherein the response of the olfactory receptor is inactivation of the olfactory receptor, and the step (A) is performed in the presence of a substance that activates the olfactory receptor.

10. The method according to any one of claims 1 to 9, wherein the olfactory receptor is used in the form of being carried on the cell.

11. The method according to any one of claims 1 to 10, wherein the cell is an animal cell.

12. The method according to any one of claims 1 to 11, wherein the response is measured by using intracellular cAMP concentration or intracellular calcium concentration as an indicator.

13. The method according to claim 12, wherein the intracellular cAMP concentration is measured by a reporter assay.

14. The method according to any one of claims 1 to 13, wherein the test substance is selected from the group consisting of foods, raw materials thereof, and fractionation products thereof.

15. The method according to any one of claims 1 to 14, wherein the olfactory receptor is selected from the group consisting of OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K13, OR4K14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5AU1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, OR5R1, OR5T1, OR5T2, OR5T3, ORSV1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4.

16. The method according to any one of claims 1 to 15, wherein the olfactory receptor is a human olfactory receptor.
